# EUROPEAN PATENT SPECIFICATION

(11) **EP 3 170 465 B1**
(45) Date of publication and mention of the grant of the patent: **07.10.2020**
(21) Application number: 14897803.4
(22) Date of filing: 17.07.2014
(51) Int. Cl.: A61B 17/68, A61F 2/40, A61B 17/72

(54) **DEVICE FOR FIXING PROXIMAL HUMERUS**
VORRICHTUNG ZUR FIXIERUNG EINES PROXIMALEN HUMERUS
DISPOSITIF DE FIXATION DE L'HUMÉRUS PROXIMAL

(43) Date of publication of application: 24.05.2017
(73) Proprietor: Tang, Peifu, Beijing 100853 (CN); Chen, Hua, Beijing 100853 (CN); Beijing Guoxietang Technology Development Co., Ltd., Bio Medicine Industry Base Daxing District Beijing 102600 (CN)
(72) Inventor: CHEN, Hua, Beijing 100853 (CN); TANG, Peifu, Beijing 100853 (CN); DUAN, Hongjie, Beijing 102600 (CN)
(74) Representative: Vidon Brevets & Stratégie
(86) International application number: PCT/CN2014/000681
(87) International publication number: WO 2016/008062

(56) References cited:
- CN-A- 101 340 851
- CN-A- 103 393 456
- CN-A- 103 800 064
- CN-A- 104 083 198
- CN-U- 204 121 146
- US-A- 5 702 486
- US-A- 5 776 194
- US-A1- 2005 187 550
- US-A1- 2007 123 878
- US-A1- 2008 009 873
- US-A1- 2009 248 025

## Description

### FIELD OF THE INVENTION

The present invention belongs to the field of medical instruments, in particular relates to a proximal humerus fixing device. The closest prior art is document US 5702486 A, which defines the preamble of claim 1.

### BACKGROUND OF THE INVENTION

Proximal humeral fractures refer to fractures in the parts above the greater tuberosity of the humerus, accounting for 4%-5% of systemic fractures, and 26% of shoulder fractures. As proximal humeral fractures are multiple, their treatment and fixing as well as the postoperative recovery of a patient are particularly important in avoiding leaving any potential problems for the patients. Moreover, proximal humeral fractures are common in the middle-aged and elderly people. As these people are normally complicated by severe osteoporosis and are susceptible to comminuted fractures, their proximal humeri are short of effective inner side support. When fixing is conducted for humeral fractured parts of such people, such problems as insufficient holding force of nails and lack of effective inner side mechanical support crop up frequently; in the long run, the phenomena that the head of the humerus introverts, and nails cut out of the head of the humerus and enter the joint often occur to a patient after treatment, which seriously affect the function of the shoulder joint of the patient, and thus make treatment extremely tricky.

Currently, proximal humeral fractures are treated with such means as locking steel plates, shoulder hemiarthroplasty and intramedullary nail fixing.

Improvements have been achieved in fixing the fractured parts with the locking steel plates utilizing the nail locking technology and the cross-locking technology. However, the effective inner side support is still absent, and there exists the risk that nails enter the joint. Shoulder hemiarthroplasty tends to cause poor heeling or absorption of the greater tuberosity of the humerus, which may lead to the loss of the moment arm with effective rotator cuff functions, thus significantly affecting the flexion and abduction functions of the shoulder joint. Although intramedullary nail fixing can effectively solve the problem of inner side support, the fixing on the head of the humerus by nails is limited, and due to the lack of stable fixing on severe comminuted fractures, the nails can't provide effective mechanical support for the early functional rehabilitation of the shoulder joint. Furthermore, a drill is needed to make entering points for the implantation of intramedullary nails, and the intramedullary nails are obviously not matched with the internal structure of the medullary cavity, which will affect intramedullary blood supplying.

Consequently, although the prior art provides a variety of solutions for the fixing treatment of proximal humeri after fracture, fixing effects are not good enough, especially for comminuted fractures, for which powerful inner side support can't be provided, and moreover, the prior art is disadvantageous to the postoperative recovery of a patient.

### SUMMARY OF THE INVENTION

In order to solve the drawbacks existing in the prior art that the inner side support for proximal humeral fractures is limited, treatment effects on comminuted fractures are poor, and the restoration of shoulder joint functions is disadvantaged, the present invention provides a proximal humerus fixing device.

The invention is defined in claim 1.

According to an embodiment of the present invention, the surface of the fixer is provided with a groove.

According to another embodiment of the present invention, a side hole is disposed on the second contact portion.

According to yet another embodiment of the present invention, the anti-rotation nails are disposed to be three in number.

According to yet another embodiment of the present invention, the tail portion of each anti-rotation nail is fixedly disposed on the fixer through a nut.

According to yet another embodiment of the present invention, the main nail, the anti-rotation nails and/or the locking nails are positioned and disposed through a guider.

According to yet another embodiment of the present invention, the materials for manufacturing the fixer comprise: metallic materials or absorbable materials.

According to yet another embodiment of the present invention, the metallic materials comprise: titanium alloys, stainless steel, tantalum and/or memory alloys.

According to yet another embodiment of the present invention, the fixer is 3 cm to 20 cm long.

The proximal humerus fixing device provided by the present invention is disposed in the medullary cavity of the proximal humerus, which realizes effective support for the head of the humerus. The upper end of the fixer has a first contact portion having a plane structure and a second contact portion in a protrusion shape; these two portions can be matched with the structures of the head of the humerus and the greater tuberosity of the humerus respectively, thereby providing effective support for the proximal humeral fractured parts. The far end of the lower end of the fixer is in a bullet shape, extends distally and does not expand the marrow, which can reduce damage caused to the blood supplying in the medullary cavity. Used in combination with the fixer, the main nail, the anti-rotation nails and the locking nails can enhance the fixing support for the proximal humeral fractured parts, and prevent the fixer from displacing and sliding off. The present fixing device has strong fixing force, eliminates the risk that nails enter the joint, and will not lead to incomplete healing of the greater tuberosity of the humerus, which may provide effective mechanical support for the early functional rehabilitation of the shoulder joint.

### BRIEF DESCRIPTION OF THE FIGURES

Through reading the detailed description of the non-limiting embodiments made with reference to the accompanying drawings, other features, purposes and advantages of the present invention will become more apparent:
Figure 1 is a structural schematic diagram of an embodiment of a proximal humerus fixing device provided in accordance with the present invention;
Figure 2 is a top view of a first contact portion of the proximal humerus fixing device provided in accordance with the present invention.

Like reference numerals designate the same or similar parts throughout the figures.

### Reference numerals:

| | | | |
|---|---|---|---|
| 1 | Fixer | 11 | First contact portion |
| 12 | Second contact portion | 13 | Lower end |
| 2 | Main nail | 3 | Anti-rotation nail |
| 4 | Locking nail | | |

### DETAILED DESCRIPTION

Many different embodiments or examples are provided in the following disclosure for implementing different structures of the present invention. In order to simplify the disclosure of the present invention, the components and dispositions of particular examples are described below. Furthermore, the present invention may repeat reference numbers and/or letters in different examples. Such repetition is for the purpose of simplicity and clarity, and the repetition itself does not indicate the relationship between the discussed various embodiments and/or dispositions. It should be noted that the illustrated components in the drawings are not necessarily drawn to scale. The present invention omits the description of known components as well as processing technology and technique so as to avoid unnecessary limitation to the present invention.

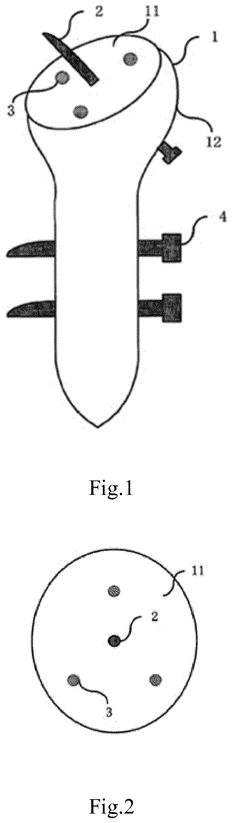

Referring to Figure 1, Figure 1 is a structural schematic diagram of an embodiment of a proximal humerus fixing device provided in accordance with the present invention.

The fixing device comprises: a fixer 1, a main nail 2, anti-rotation nails 3 and locking nails 4. During use, the fixer 1 is disposed in a medullary cavity of the proximal humerus and is matched with the medullary cavity of the proximal humerus. In order to reduce damage caused to the blood supplying in the medullary cavity, the surface of the fixer 1 may be provided with a groove.

The upper end of the fixer 1 comprises a first contact portion 11 and a second contact portion 12, wherein the first contact portion 11 is in contact with a head of the humerus to support the head of the humerus. The first contact portion 11 is of a plane structure so as to have a bigger contact area with the head of the humerus. In order to better support the head of the humerus and have it disposed firmly in the medullary cavity, the first contact portion 11 is preferably an elliptical plane. According to the position of the head of the humerus on the humerus and the physiological structural relationship between the head of the humerus and the medullary cavity, the first contact portion 11 is disposed at a certain angle with respect to the medullary cavity so as to achieve the optimum supporting force. Preferably, the angle is 45 degrees to 50 degrees, such as 45 degrees, 48 degrees or 50 degrees.

The second contact portion 12 is in contact with a greater tuberosity of the humerus, and in order to be matched with the structure of the greater tuberosity of the humerus, the second contact portion 12 is in a protrusion shape. Preferably, a side hole is disposed on the second contact portion 12, and when suture fixing is required, it can be realized through this side hole.

The far end of the lower end of the fixer 1 is in a bullet shape, which is not in contact with the medullary cavity. The streamlined design reduces sharp contact with the medullary cavity, avoids excessive concentration of stress and reduces damage caused to the marrow in the medullary cavity.

Preferably, the materials for manufacturing the fixer 1 comprise: metallic materials or absorbable materials, wherein the metallic materials include, but are not limited to: titanium alloys, stainless steel, tantalum and/or memory alloys. When memory alloys are adopted, the fixer 1 is implanted in vitro, and under the state of body temperature, the medullary cavity of the proximal humerus is filled after rewarming so as to realize the support for the head of the humerus.

Preferably, the length of the fixer 1 varies depending on the differences in the height of users, bone length, humerus state, fracture degree, osteoporosis degree and so on, and preferably, the fixer 1 is 3 cm to 20 cm long, such as 3 cm, 10 cm or 20 cm. According to the physiological state of ordinary patients, the fixer 1 is preferably 8 cm long.

The main nail 2 is used for fixing the head of the humerus and the first contact portion 11. The head of the humerus is pulled to the first contact portion 11 of the fixer 1.

The head of each anti-rotation nail 3 enters the head of the humerus, and the tail portion of each anti-rotation nail 3 is fixedly connected with the fixer 1. Preferably, the tail portion of each anti-rotation nail 3 is fixedly disposed on the fixer 1 through a nut. The anti-rotation nails 3 is used for fixing the head of the humerus so as to prevent the head of the humerus from rotating on the first contact portion 11 of the fixer 1. As the stability of the triangular structure is the highest, the anti-rotation nails 3 are disposed to be three in number. Preferably, the arrangement of the anti-rotation nails is triangular, as shown in Figure 2. More preferably, the main nail 2 is located in the center of the triangle formed by the anti-rotation nails 3. The anti-rotation nails 3 have no threads and allow partial absorption of the head of the humerus. After the partial absorption of the head of the humerus, the contact of the head of the humerus with the first contact portion 11 will be closer, and thus the supporting effects of the fixer 1 on fractured parts will be better. The length of each anti-rotation nail 3 changes as patient conditions vary, and it is advisable that the length should not be so long that the anti-rotation nails 3 can penetrate into the joint.

The locking nails 4 are disposed on the lower end of the fixer 1 and are used for enabling the fixer 1 and the humeral shaft to be fixedly connected with each other.

In order to reduce damage caused to the blood supplying in the medullary cavity and make the fixer 1 contact the medullary cavity to realize stable fixing, the cross-section of the proximal end of the fixer 1 is disposed to be triangular. It should be noted that the proximal end of the fixer 1 refers to a portion between the locking nails 4 adjacent to the upper end of the fixer 1 and the first contact portion 11.

Preferably, in order to facilitate accurate positioning of various nails during surgical operation, the main nail 2, the anti-rotation nails 3 and/or the locking nails 4 all are positioned and disposed through a guider.

Preferably, the fixing device provided by the present invention may also be used in combination with an extramedullary fixing device so as to further enhance fixing effects.

During the practical application of the fixing device provided by the present invention, a patient needs to be in a beach chair position or a horizontal position, conventional disinfection is conducted, and a sterile towel is paved; thereafter, the deltoid-pectoralis major interscalene approach is adopted to expose the proximal humeral fractured part of a patient.

After the fractured part is exposed, the implantation operation of the fixing device begins. First, a distraction tong is implanted into the fracture void (namely, between the head of the humerus and the humeral shaft) for distraction, the length of the inner side of the proximal humerus is recovered, and a Kirschner's wire is used to fix the head of the humerus on the scapula glenoid temporarily (to maintain the collodiaphysial angle of the proximal humerus to be 130 degrees to 150 degrees); then, fractured blocks of the greater and lesser tuberosities are opened up, and the humeral shaft is pulled outwardly to expose the medullary cavity of the proximal humerus; thereafter, the elbow joint is kept to be forward, the fixing device is inclined backward by 20 degrees to 40 degrees, and the intramedullary dissection fixing device is implanted to an appropriate depth to reduce fractures; under the fluoroscopic monitoring, the head of the humerus is made to be in close contact with the first contact portion 11; the main nail 2 is implanted through the extramedullary guider connected on the fixing device 1, and pulls the head of the humerus to be in close contact with the first contact portion 11; afterwards, the anti-rotation nails 3 and the locking nails 4 at the far end of the fixing device 1 are implanted through the guider (to enable the steel plates and the humeral shaft to be connected together); after the main nail 2, the anti-rotation nails 3 and the locking nails 4 are all implanted, the extramedullary guider is removed; eventually, the greater and lesser tuberosities are fixed on the main nail 2 or the second contact portion 12 through non-absorbable sutures or steel wires in the manner of tension bands. As the second contact portion 12 is provided with a side hole, suture or steel wire fixing can be realized through the side hole, with better fixing effects.

The fixing device provided by the present invention directly enters the medullary cavity from between the fractured greater and lesser tuberosities and is matched with the medullary cavity of the proximal humerus. The first contact portion 11 is disposed at a certain angle to support the head of the humerus, and the protrusion of the second contact portion 12 supports the greater tuberosity; the fixing and anti-rotation of the head of the humerus is realized through the main nail 2 and the anti-rotation nails 3. The transmission of the supporting force of the main nail 2 allows the loosening of the main nail 2 to realize sliding pressurization. A side hole is disposed on the fixer 1, and the side hole allows ingrowth of new bones. The length of each anti-rotation nail 3 is appropriate, and after bone absorption, the anti-rotation nails 3 will not penetrate into the shoulder joint to cause damage thereto. The fixer 1 is matched with the medullary cavity to realize the inner side support of the proximal humerus. The fixing device of the present invention may realize the connection between the head of the humerus and the humeral shaft and thus restore mechanical support. Bone tissues heal as they wrap the fixer 1, which realizes the effect similar to that of reinforcing steel bar and cement injection, thus having strong fixing force.

Although the exemplary embodiments and advantages thereof have been described in detail, it should be appreciated that various changes, alternatives and modifications may be made to these embodiments without departing from the scope of the present invention as defined by the appended claims. For other examples, those of ordinary skill in the art will readily appreciate that the order of process steps may be changed while being maintained within the scope of the present invention.

Moreover, the application range of the present invention is not limited to the processes, mechanisms, manufacturing, material composition, means, methods and steps of particular embodiments described in the description. Those of ordinary skill in the art will readily appreciate from the disclosure of the present invention that for the processes, mechanisms, manufacturing, material composition, means, methods or steps that already exist at present or will be developed in the future, which perform the functions substantially identical to those performed by the corresponding embodiments described in the present invention or achieve substantially identical results, they may be applied according to the present invention. Therefore, the appended claims of the present invention are intended to include such processes, mechanisms, manufacturing, material composition, means, methods or steps within its scope.

## Claims

1. A proximal humerus fixing device, wherein the fixing device comprises: a fixer (1), a main nail (2), anti-rotation nails (3) and locking nails (4); wherein the fixer is adapted to be disposed in a medullary cavity of a proximal humerus and is matched with the medullary cavity of the proximal humerus, the fixer having an axis that is configured to be parallel with an axis of the medullary cavity when the fixer is disposed therein; an upper end of the fixer comprising a first contact portion (11) and a second contact portion (12); wherein the first contact portion is adapted to contact a head of the humerus and is of a plane structure; wherein the second contact portion is adapted to contact a greater tuberosity of the humerus and has a protrusion shape; wherein a far end of a lower end (13) of the fixer has a bullet shape, and the lower end of the fixer is intended not to contact the medullary cavity; wherein the main nail is adapted for fixing the head of the humerus to the first contact portion; wherein a head of each anti-rotation nail is adapted to enter the head of the humerus, and a tail portion of each anti-rotation nail is adapted to be fixedly connected to the fixer; wherein the locking nails are disposed on the lower end of the fixer and are for enabling the fixer and the proximal humerus to be fixedly connected to each other, **characterised in that** the first contact portion is disposed at an angle of 45-50 degrees with respect to the axis of the fixer.

2. The fixing device of claim 1, wherein the surface of the fixer is provided with a groove.

3. The fixing device of claim 1 or 2, wherein a side hole is disposed on the second contact portion.

4. The fixing device of any one of claims 1-3, wherein the fixing device comprises three anti-rotation nails

5. The fixing device of claim 1, wherein the tail portion of each anti-rotation nail is adapted to be fixedly disposed on the fixer through a nut.

6. The fixing device of claim 1, wherein the main nail, the anti-rotation nails and/or the locking nails are configured to be positioned and disposed through a guider.

7. The fixing device of any one of claims 1-6, wherein the the fixer is manufactured from a material selected from: metallic materials or absorbable materials.

8. The fixing device of claim 7, wherein the metallic materials comprise: titanium alloys, stainless steel, tantalum and/or memory alloys.

9. The fixing device of any one of claims 1-8, wherein the fixer has a length of between 3 cm and 20 cm.

## Patentansprüche

1. Proximale Humerus-Fixiervorrichtung, wobei die Fixiervorrichtung umfasst: einen Fixierer (1), einen Hauptnagel (2), Antirotationsnägel (3) und Verriegelungsnägel (4); wobei der Fixierer angepasst ist, um in einer Markhöhle eines proximalen Humerus angeordnet zu werden und der an die Markhöhle des proximalen Humerus angepasst ist, wobei der Fixierer eine Achse aufweist, die eingerichtet ist, parallel mit einer Achse der Markhöhle zu sein, wenn der Fixierer darin angeordnet ist; ein oberes Ende des Fixierers umfasst einen ersten Kontaktabschnitt (11) und einen zweiten Kontaktabschnitt (12); wobei der erste Kontaktabschnitt angepasst ist, einen Kopf des Humerus zu kontaktieren, und der eine ebene Struktur aufweist; wobei der zweite Kontaktabschnitt angepasst ist, um eine größere Tuberositas des Humerus zu kontaktieren, und der eine Vorsprungsform aufweist; wobei ein entferntes Ende eines unteren Endes (13) des Fixierers eine Kugelform aufweist, und das untere Ende des Fixierers vorgesehen ist, die Markhöhle nicht zu kontaktieren; wobei der Hauptnagel angepasst ist, den Kopf des Humerus am ersten Kontaktabschnitt zu fixieren; wobei ein Kopf jedes Anti-Rotationsnagels angepasst ist, in den Kopf des Humerus einzutreten, und ein Ausläuferabschnitt jedes Anti-Rotationsnagels angepasst ist, fest mit dem Fixierer verbunden zu werden; wobei die Verriegelungsnägel an dem unteren Ende des Fixierers angeordnet sind und dafür sind, um zu ermöglichen den Fixierer und den proximalen Humerus fest miteinander zu verbinden, **dadurch gekennzeichnet, dass** der erste Kontaktabschnitt mit einem Winkel von 45-50 Grad bezogen auf die Achse des Fixierers angeordnet ist.

2. Fixiervorrichtung nach Anspruch 1, wobei die Oberfläche des Fixierers mit einer Nut bereitgestellt ist.

3. Fixiervorrichtung nach Anspruch 1 oder 2, wobei ein Seitenloch am zweiten Kontaktabschnitt angeordnet ist.

4. Fixiervorrichtung nach einem der Ansprüche 1-3, wobei die Fixiervorrichtung drei Anti-Rotationsnägel umfasst.

5. Fixiervorrichtung nach Anspruch 1, wobei der Ausläuferabschnitt jedes Anti-Rotationsnagels angepasst ist, fest am Fixierer durch eine Mutter angeordnet zu werden.

6. Fixiervorrichtung nach Anspruch 1, wobei der Hauptnagel, die Anti-Rotationsnägel und/oder die Verriegelungsnägel eingerichtet sind, dass sie durch eine Führung positioniert und angeordnet werden können.

7. Fixiervorrichtung nach einem der Ansprüche 1-6, wobei der Fixierer aus einem Material hergestellt ist, ausgewählt von: metallischen Werkstoffen oder absorbierbaren Materialien.

8. Fixiervorrichtung nach Anspruch 7, wobei die metallischen Materialien umfassen: Titanlegierungen, Edelstahl, Tantal und/oder Memory-Legierungen.

9. Fixiervorrichtung nach einem der Ansprüche 1-8, wobei der Fixierer eine Länge zwischen 3 cm und 20 cm aufweist.

## Revendications

1. Dispositif de fixation de l'humérus proximal, dans lequel le dispositif de fixation comprend :
un fixateur (1), un clou principal (2), des clous anti-rotation (3) et des clous de verrouillage (4) ;
dans lequel le fixateur est adapté pour être installé dans une cavité médullaire d'un humérus proximal et est apparié à la cavité médullaire de l'humérus proximal, le fixateur ayant un axe qui est configuré pour être parallèle à un axe de la cavité médullaire lorsque le fixateur y est installé ; une extrémité supérieure du fixateur comprenant une première partie de contact (11) et une seconde partie de contact (12) ;
dans lequel la première partie de contact (11) est adaptée pour entrer en contact avec une tête de l'humérus et présente une structure plane ;
dans lequel la seconde partie de contact est adaptée pour entrer en contact avec une tubérosité plus grande de l'humérus et présente une forme en saillie ;
dans lequel une extrémité distale d'une extrémité inférieure (13) du fixateur présente une forme fuselée, et il est prévu que l'extrémité inférieure du fixateur n'entre pas en contact avec la cavité médullaire ;
dans lequel le clou principal est adapté pour fixer la tête de l'humérus à la première partie de contact ;
dans lequel une tête de chaque clou anti-rotation est adaptée pour pénétrer dans la tête de l'humérus, et une partie queue de chaque clou anti-rotation est adaptée pour être reliée de manière fixe au fixateur ;
dans lequel les clous de verrouillage sont disposés sur l'extrémité inférieure du fixateur et sont prévus pour permettre au fixateur et à l'humérus proximal d'être reliés de manière fixe l'un à l'autre,
**caractérisé en ce que** la première partie de contact est disposée à un angle de 45 à 50 degrés par rapport à l'axe du fixateur.

2. Dispositif de fixation selon la revendication 1, dans lequel la surface du fixateur est pourvue d'une rainure.

3. Dispositif de fixation selon la revendication 1 ou 2, dans lequel un orifice latéral est disposé sur la seconde partie de contact.

4. Dispositif de fixation selon l'une quelconque des revendications 1 à 3, dans lequel le dispositif de fixation comprend trois clous anti-rotation.

5. Dispositif de fixation selon la revendication 1, dans lequel la partie queue de chaque clou anti-rotation est adaptée pour être disposée de manière fixe sur le fixateur par l'intermédiaire d'un écrou.

6. Dispositif de fixation selon la revendication 1, dans lequel le clou principal, les clous anti-rotation et/ou les clous de verrouillage sont configurés pour être positionnés et disposés par l'intermédiaire d'un élément de guidage.

7. Dispositif de fixation selon l'une quelconque des revendications 1 à 6, dans lequel le fixateur est fabriqué à partir d'un matériau sélectionné parmi des matériaux métalliques ou des matériaux absorbables.

8. Dispositif de fixation selon la revendication 7, dans lequel les matériaux métalliques comprennent : des alliages de titane, l'acier inoxydable, le tantale et/ou des alliages à mémoire.

9. Dispositif de fixation selon l'une quelconque des revendications 1 à 8, dans lequel le fixateur a une longueur entre 3 cm et 20 cm.
